# EUROPEAN PATENT APPLICATION

(11) **EP 0 870 514 A1**
(43) Date of publication of application: **14.10.1998**
(21) Application number: 97105921.7
(22) Date of filing: 10.04.1997
(51) Int. Cl.: A61L 27/00, A61F 5/445

(54) **Liquid absorbent material used in a pouch for a stoma**

(71) Applicant: Komine, Keiji, Funabashi-shi, Chiba (JP); Ishigaki, Ryuzo, Koshigaya-shi, Saitama (JP)
(72) Inventor: Komine, Keiji, Funabashi-shi, Chiba (JP); Ishigaki, Ryuzo, Koshigaya-shi, Saitama (JP)
(74) Representative: Stachow, Ernst-Walther, Prof. Dr.

(57) **Abstract**

A liquid absorbent material to be used in a pouch for a stoma wherein a powdery synthetic polymeric highly water absorbing resin is filled in a sealed state in a water soluble or water dispersible tube having an outer diameter that can be inserted from a urine discharging tube into a bag of a pouch for a urinary tract stoma and a length that can be contained in the bag, and formed into a stick-shape. Liquid excrements flowing out of the stoma into the bag of the pouch can be rapidly solidified into jelly by the liquid absorbent material, thereby reliably preventing liquid excrements or bad smells thereof from leaking through the attaching port of the pouch or the like and preventing liquid excrements from deposition to the adhesives used for securing the pouch to the abdominal wall which would lead to deterioration of tackiness of the adhesives, to enable repeating use of the stoma pouch.

## Description

### BACKGROUND OF THE INVENTION

### Field of the Invention

The present invention concerns a liquid absorbent material to be used in a pouch for a stoma attached to a discharging port of a digestive tract stoma (artificial anus) or urinary tract (artificial bladder).

Stoma pouches include those attached to a discharging port of a digestive tract stoma prepared by leading out a cut end of an intestine, from which a lesional part is excised by operation, through an aperture perforated in an abdominal wall and sutured to the abdominal wall and those attached to a discharging port of a urinary tract stoma prepared by perforating an aperture through an abdominal wall for discharging urine sent from a kidney to the outside of a body.

The pouches of these types are adapted to receive feces or urine discharged occasionally and involuntarily from a stoma, which can not control discharge by the own will of a wearer, into a bag such as a vinyl plastic bag for disposal and administration. The bag has an attaching port having a hole as a receptacle of excrements flowing out of the stoma and a flange disposed at the periphery of the hole, and the bag is attached by fixing the attaching port to the abdominal wall and suspending the bag from the abdominal wall.

The method of fixing the attaching port of the pouch to the abdominal wall includes coating adhesives to the flange of the attaching port and appending the flange directly to the skin of the abdominal wall, appending to a face plate fixed previously by adhesives to the skin of the abdominal wall, attaching a packing ring for prevention of liquid leakage to the flange of the attaching port and fitting the packing ring to an annular rib protruded from the face plate.

Further, the pouch for the urinary tract stoma has a urine discharging tube as a drain disposed at the bottom of a bag for flowing out urine. The urine discharging tube is formed with a fine and elongate soft plastic tube having about 5 to 6 mm inner diameter and about 70 to 80 mm length. The pouch also has a drain stopper such as a cap that covers the top end for closing the tract or a clip for collapsing and closing the tubular tract.

The pouch for the digestive tract stoma includes a tightly closed disposable type with no drain which is discarded after the use only for once and a lower open type having a drain aperture perforated at the bottom of the bag for repeating use. The lower open type pouch has a clip stopper for closing the drain aperture.

The drain is disposed to decrease the frequency of exchanging the pouch thereby moderating economical burden or troublesome labor on an ostomate but, actually, the pouch with the drain is not used repeatingly so often.

One of major reasons is that excrements and bad smells thereof are present and remain on the surface of the bag, even after excrements accumulated in the bag are discharged from the drain, and gradually increase bad smells with elapse of time. What is most anxious to the ostomate is leakage of excrements and bad smells thereof from the pouch. Since bad smells of feces are anxious particularly in the pouch for the digestive tract stoma, even the lower open type pouch having the drain aperture were discard so far without repeating use.

The lower open type pouches for the digestive tract stoma, in which a smooth deodorizing film for making feces or bad smells thereof less depositable on the inside of the pouch or in which a powdery or tablet deodorant is incorporated in the bag are also commercially available.

However, even the smooth deodorizing film can not completely discharge feces in the bag through the drain aperture but can provide only such an extent of effect as somewhat decreasing the amount of feces deposited and remained in the bag.

Further, since the deodorant is lost together with discharge of feces through the drain aperture, the deodorizing effect is not long lasting.

Further, the powdery deodorant, if it is incorporated into the pouch as it is, may possibly deposit to the stoma and bring about a worry of causing skin eruption or inflammation to muscosa of the stoma if the deodorant is a chemical substance.

Further, a tablet deodorant, if incorporated into the pouch for urinary tract stoma, may possibly clog the urine discharge tube thus hindering smooth discharge of urine.

Then, another reason of hindering the repeating use of the pouch having the drain is that the water content in the excrements accumulated in the bag of the pouch adheres and penetrates into adhesives used for appending the attaching port to skins or a face plate and deteriorate the tackiness of the adhesives tending to cause peeling of the attaching port and making it impossible to secure the pouch to the abdominal wall.

Particularly, liquid excrements such as diarrhea feces or urine shake or more in the bag of the pouch tending to adhere to and penetrate into the adhesives, and they shake pulsatively during walking or movement to dissolve the adhesives and rapidly lower the tackiness, so that this may cause a worry of dropping the pouch attached to the stoma from a abdominal wall in an early stage.

By the way, excrements accumulated in the bag are disposed by flashing, for example, to a toilet stool through the drain in a case of a pouch with drain and through the attaching port in a case of a pouch with no drain. However, such treatment is extremely troublesome and, if it is done carelessly, diarrhea feces or urine pushed out of the drain or the attaching port are scattered around to possibly contaminate an ostomate's body or garment, or soil the stool or floor surface of the toilet.

Accordingly, it was proposed an invention of disposing a bag of a water soluble paper having a powdery water absorbing resin or a solid block water absorbing resin for gelling watery or soft feces in the bag of a pouch for digestive tract stoma having no drain aperture, to facilitate the disposable of excrements (refer to Japanese Patent Laid-Open Hei 5-175950).

However, the invention has no concerns at all for rapid gelling of excrements regarding the water absorbing resin incorporated in the bag of the pouch, since the invention was proposed perhaps with an aim of facilitating the treatment upon detaching the pouch from the abdominal wall and flashing out the excrements accumulated in the bag through the attaching port.

That is, since the powder of the water absorbing resin sealed in the bag is densely disposed in the bag, it is less disintegratable and takes much time till the entire portion is dissolved in the liquid of the excrements. The solid block water absorbing resin takes a further time. If the resin has a volume and a size corresponding to the volume of the pouch it takes further longer time.

Although the excrements may be entirely gelled till the pouch is detached from the abdominal wall and the excrements accumulated in the bag are disposed, it is impossible, soon after the attaching of the pouch to the stoma, to rapidly gel the liquid excrements flowing out in a great amount from the discharging port of the stoma without shaking them in the bag of the pouch. Accordingly, this prior invention can not overcome the problem that excrements or the bad smells thereof should leak through the attaching port of the pouch attached to the stoma by the shaking of the liquid or excrements adhered to the adhesives used for securing the attaching port of the pouch to the abdominal wall thereby deteriorating the tackiness of the adhesives.

Further, this prior invention relates to a disposable pouch for digestive tract stoma with no drain aperture, so that the pouch can not be used again after the attaching port is once peeled from the abdominal wall and the excrements are discharged through the attaching port thereof since the adhesives bonding the attaching port to the abdominal wall lose the tackiness and have to be discarded. Therefore, this prior invention has no idea of supplementing the water absorbing resin in the bag of the pouch after discharging the excrements.

Further, in the invention described above, a bag having a powder of the water absorbing resin sealed therein or the solid block of water absorbing resin can not but be inserted into the bag through the attaching port of the pouch. However absorbent products exclusively used for this purpose having such a small size that can be inserted as they are through the attaching port have not yet been commercially available.

### OBJECT OF THE INVENTION

It is an object of the present invention to provide a liquid absorbent material used exclusively for a pouch which can be supplemented easily by a required amount into a bag of a stoma pouch having a drain with the pouch being secured to the abdominal wall and attached to the stoma for enabling the pouch to be used over and over always in a comfortable condition.

Another object of the present invention is to enable liquid excrements flowing out of the stoma into the bag of the pouch to be gelled rapidly by the liquid absorbent material.

### SUMMARY OF THE INVENTION

The foregoing object of the present invention can be attained by a liquid absorbent material to be used in a pouch for a stoma wherein a powdery synthetic polymeric highly water absorbing resin is filled in a tightly sealed state in a water soluble or water dispersible tube having an outer diameter that can be inserted from a urine discharging tube into a bag of a pouch for a urinary tract stoma and a length that can be contained in the bag, and formed into a stick-shape.

The stick-shape liquid absorbent material can be inserted collectively by a required number corresponding to the volume of the pouch also for a disposable digestive tract stoma with no drain from the attaching port into the bag of the pouch, as well as may be inserted also for a pouch for a urinary tract stoma having a urine discharging tube or a pouch for a digestive tract stoma having a drain aperture from an attaching port into a bag of such pouch upon use for the first time.

Then, when the pouch with drain is used at or after the second time, a required number of the stick-shape liquid absorbent materials is inserted and supplemented from the drain into the bag of the pouch.

The stick-shape liquid absorbent material according to the present invention can be inserted easily also from a fine urine discharging tube of a pouch for a urinary tract stoma, and can be inserted collectively by several sticks from the drain aperture of the pouch for the digestive tract stoma.

As described above, when the stick-shape liquid absorbent materials supplemented by the required number in the bag of the pouch, the tube constituting the outer shell is rapidly dissolved and dispersed in the liquid of excrements flowing out of the stoma and the powdery synthetic polymeric highly water absorbing resin filled in the tube swells by absorbing the water content of the excrements in a not releasable manner and gels the liquid excrements and solidifies them into jelly.

The total area of the liquid excrements flowing out of the discharging port of the stoma that can be in contact with the liquid absorbent material supplemented in the bag of the pouch is extremely large corresponding to the sum of the surface area of the tube constituting the outer shell for each of the liquid absorbent materials, and each of the liquid absorbent materials is present being distributed at random in the bag of the pouch. Accordingly, the liquid of the excrements flowing out of the discharging port of the stoma is uniformly brought into contact with the liquid absorbent materials. Further, since the tube of the liquid absorbent material has such a fine elongate shape as can be inserted into the urine discharging tube of the pouch for the urinary tract stoma, the powder of the synthetic polymeric highly water absorbing resin filled in the tube is highly disintegratable, and the powder is disintegrated and released thoroughly from the inside of the tube upon dissolution and dispersion of the tube into the liquid of excrements, to rapidly gel the liquid excrements.

Accordingly, it is possible to prevent that the liquid excrements flowing out of the discharging port of the stoma from shaking in the bag of the pouch, thereby leaking the excrements and the bad smells thereof to the outside through the attaching port of the pouch, or to prevent the attaching port from dropping out of the abdominal wall by the deposition of the liquid of the excrements to the adhesives used for securing the attaching port to the abdominal wall.

Since the excrements gelled and solidified into jelly less scatter around upon disposal by discharging them through the urine discharging tube of the pouch for the urinary tract stoma or the drain aperture of the pouch for the digestive tract stoma or the disposal is facilitated and the amount of the excrements deposited and remained in the bag of the pouch is decreased.

Further, since the liquid absorbent material is fine and the outer shell of the material is formed of a water soluble or water dispersible tube which is easily dissolved and dispersed in the liquid, there is no worry that the material clogs the urine discharging tube of the pouch for the urinary tract stoma when urine is discharged from the urine discharging tube and disposed.

Further, while the powder of the synthetic polymeric highly absorbing resin used for the liquid absorbent material in the present invention sometimes has a spiny surface on the particle and when the powder is inserted as it is in the pouch, it may possibly cause pinholes in the bag or deposition of powder to muscosa of the stoma, resulting in skin eruption or inflammation. However, such a worry can be eliminated by filling the powdery resin in a sealed state in the tube as described above.

### BRIEF DESCRIPTION OF THE DRAWINGS

Fig. 1 is a view illustrating a liquid absorbent material of a first embodiment according to the present invention;
Fig. 2 is a view illustrating a cross sectional structure of the liquid absorbent material in an enlarged scale;
Fig. 3 is a view illustrating the state of using the liquid absorbent material;
Fig. 4 is a view illustrating a liquid absorbent material of a second embodiment according to the present invention; and
Fig. 5 is a view illustrating a cross sectional structure of the liquid absorbent material in an enlarged scale.

### DESCRIPTION OF PREFERRED EMBODIMENTS

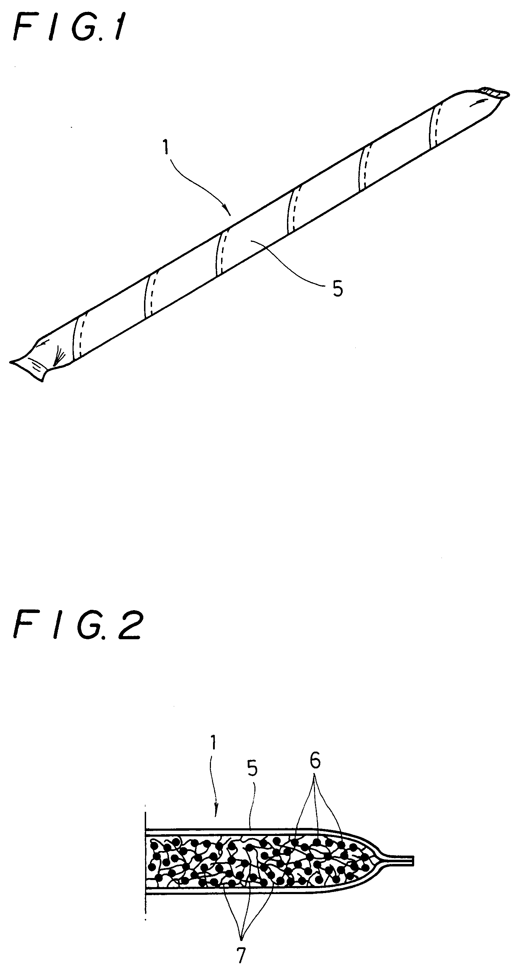

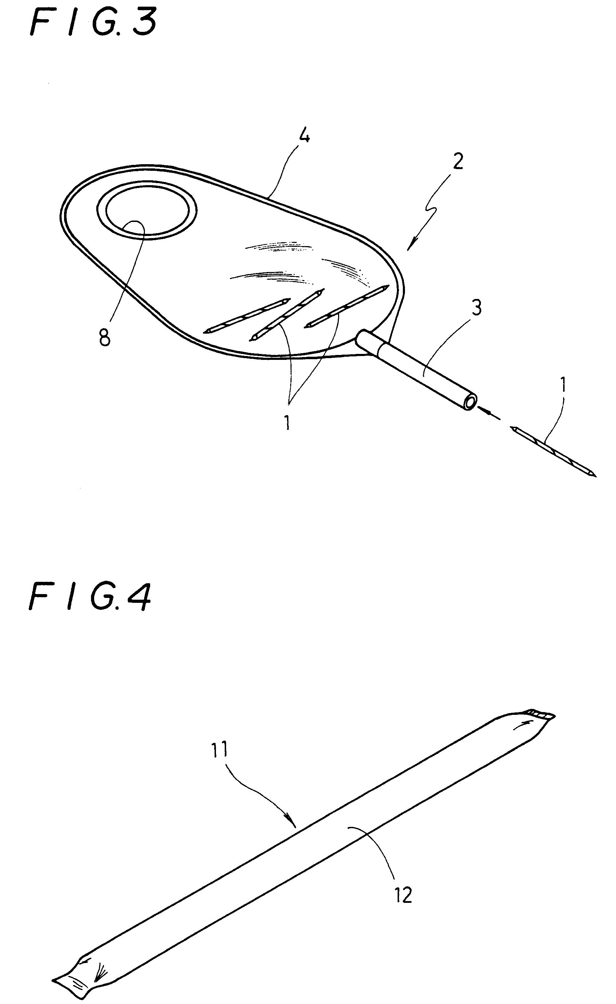

A liquid absorbent material 1 used in a pouch for a stoma shown in Fig. 1 and Fig. 2 is formed into a stick-shape by filling a powdery synthetic polymeric highly water absorbing resin 6 having a property of swelling by water absorption in a sealed state into a water soluble or water dispersible tube 5 having an outer diameter that can be inserted from a urine discharging tube 3 to a bag 4 of a pouch 2 for a urinary tract stoma shown in Fig. 3 and such a length that can be contained in the bag 4.

The tube 5 is formed by spirally winding a carboxymethyl type water soluble paper tape prepared by integrating fibrous carboxymethyl cellulose formed by carboxymethylating natural cellulose fibers of 0.8 to 1.5 mm fiber length with water soluble adhesives, bonding overlaps of the wound with water soluble adhesives into a cylindrical shape having an outer diameter of 5 mm or less and a length of about 6 to 8 mm, filling the powdery synthetic polymeric highly water absorbing resin 6 to the inside of the cylinder, and then closing both ends by collapse thereby sealing the synthetic polymeric highly water absorbing resin 6.

Powder of polyacrylate highly water absorbing resin for providing gelled excrements with dry filling is used for the powdery synthetic polymeric highly water absorbing resin 6 to be filled in the tube 5 to which fibrous cellulosic highly water absorbing resin 7 is mixed at 2;1 - 4:1 weight ratio.

Fibrous caboxymethyl cellulose of 0.8 to 1.5 mm fiber length which entirely dissolves by gelling under swelling in water is used as the cellulosic highly water absorbing resin 7.

The stick-shape liquid absorbent material 1 having the foregoing constitution can be inserted easily into the bag 4 through a urine discharging tube 3 having about 5 to 6 mm diameter disposed to the pouch 2 for the urinary tract stoma, and can be inserted into the bag 4 of the pouch for urinary tract stoma by the number corresponding to the volume of the bag by properly adjusting the number of the sticks to be inserted.

When the pouch 2 for the urinary tract stoma is used for the first time, the sticks can be inserted collectively on every several sticks from the attaching port 8.

When the stick-shape liquid absorbent material 1 is thus inserted into the bag 4 of the pouch 2 for the urinary tract stoma, the tube 5 formed of the water soluble paper is dissolved and dispersed by urine flowing out of the discharging port of the stoma, and the powdery synthetic polymeric highly water absorbing resin 6 and the fibrous cellulosic highly water absorbing resin 7 filled in the tube swell and dissolve upon contact with urine, and cause urine to gel and solidify into jelly.

In this case, since the tube 5 is formed of the water soluble paper using the fibrous carboxymethyl cellulose, the tube is dissolved and dispersed in a short period of time of within about 5 to 20 sec.

Further, since the powdery synthetic polymeric highly water absorbing resin 6 filled in the fine tube 5 having a diameter of 5 mm or less is easily disintegrated and released from the inside of the tube 5 and since the fibrous cellulosic highly water absorbing resin 7 is mixed therewith, it is further disintegratable. In addition, since the fibrous cellulosic highly water absorbing resin 7 has a nature of rapidly dissolving when immersed in water with no mechanical stirring and urine is caused to penetrate uniformly and rapidly by the capillary phenomenon between each of the fibers, the dissolving and swelling rate of the highly water absorbing resins 6 and 7 are extremely high and urine flowing out of the discharging port of the stoma can be gelled rapidly.

Further, since the stick-shape liquid absorbent materials 1 inserted in the bag 4 of the pouch 2 for the urinary tract stoma are present being scattered at random in the bag 4 and the total surface area of the liquid absorbent materials 1 is large, they can uniformly gel the urine flowing out of the discharging port of the stoma.

Accordingly, it is possible to prevent the urine flowing out of the discharging port of the stoma from shaking in the bag 4 of the pouch 2 thereby leaking to the outside through the attaching port 8, or to prevent the attaching port 8 from dropping out of the abdominal wall by the deposition of urine to the adhesives used for securing the attaching port 8 to the abdominal wall.

Further, the polyacrylate highly water absorbing resin used as the powdery synthetic polymeric highly water absorbing resin 6 has a spiny surface on the particle. However, when the powdery resin is filled in a sealed state within the tube 5, there is no worry of resulting in pinholes, etc. to the bag 4 when the liquid absorbent material 1 is inserted into the bag 4 of the pouch 2 and the powdery resin deposits to the stoma before dissolved and swollen by urine which may cause skin eruption or inflammation to ostomate's muscosa.

Further, the liquid absorbent material 1 according to the present invention has a merit capable of being used both for the pouch for the urinary tract stoma and the pouch for the digestive tract stoma.

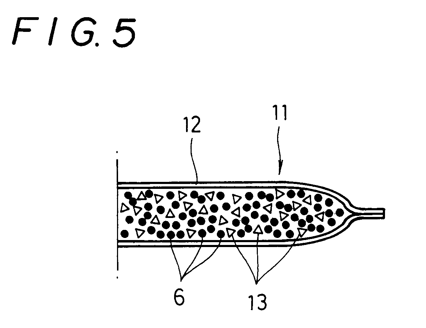

Then, a liquid absorbent material 11 used in a pouch for a stoma shown in Fig. 4 and Fig. 5 is prepared by forming a cylindrical tube 12 having an outer diameter of 5 mm or less and a length of about 6 to 8 mm with a water soluble resin film such as a water soluble vinylic film, filling a powdery synthetic polymeric highly water absorbing resin 6 and a powdery deodorant 13 mixed therewith into the tube 12 and then collapsing the both ends of the tube 12 and closing them by thermal fusion.

As the deodorant 13, green tea extracts prepared by extracting leaves of Japanese tea into hot water, purified with ethanol and then drying into a powdery form (green tea extracts MF manufactured by Maruzen Seiyaku Co.) are used. The green tea extracts are particularly suitable as the deodorant for the stoma pouch since the extracts are harmless to human bodies, cause no worry of skin eruption or inflammation to the ostomate's muscosa if they should deposit thereto and since the green tea extracts have an effect of deodorizing ammonia, for example, of lowering the ammonia residue to 3% by adding 1% extracts to a liquid containing 75 ppm of ammonia.

The powdery deodorant 13 may be mixed in the tube 5 of the liquid absorbent material shown in Fig. 1 and Fig. 2, or the fibrous cellulosic highly water absorbing resin 7 may be mixed in the tube 12 of the liquid absorbent material 11 shown in Fig. 4 and Fig. 5.

## Claims

1. A liquid absorbent material to be used in a pouch for a stoma wherein a powdery synthetic polymeric highly water absorbing resin is filled in a tightly sealed state in a water soluble or water dispersible tube having an outer diameter that can be inserted from a urine discharging tube into a bag of a pouch for a urinary tract stoma and a length that can be contained in the bag, and formed into a stick-shape.

2. A liquid absorbent material to be used in a pouch as defined in claim 1, wherein the tube is formed of water soluble paper prepared by integrating fibrous carboxymethyl cellulose with water soluble adhesives.

3. A liquid absorbent material to be used in a pouch as defined in claim 1, wherein the tube is formed of water soluble resin.

4. A liquid absorbent material to be used in a pouch as defined in any one of claims 1 to 3, wherein a fibrous cellulosic highly water absorbing resin is mixed with the synthetic polymeric highly water absorbing resin.

5. A liquid absorbent material to be used in a pouch as defined in any one of claims 1 to 4, wherein a powdery deodorant is mixed with the synthetic polymeric highly water absorbing resin.
